# FASCICULE DE BREVET EUROPEEN

(11) **EP 4 149 654 B1**
(45) Date de publication et mention de la délivrance du brevet: **02.07.2025**
(21) Numéro de dépôt: 21722891.5
(22) Date de dépôt: 04.05.2021
(51) Int. Cl.: B01D 53/00, B01D 53/22, C10L 3/10, F25J 3/02, B01D 53/30

(54) **INSTALLATION ET PROCÉDÉ DE PRODUCTION DE BIOMÉTHANE AVEC PERTE DE MÉTHANE LIMITÉE ET EMISSION DE CO2 LIMITÉE**
ANLAGE UND VERFAHREN ZUR HERSTELLUNG VON BIOMETHAN MIT BEGRENZTEM METHANVERLUST UND BEGRENZTEN CO2-EMISSIONEN
FACILITY AND METHOD FOR PRODUCING BIOMETHANE WITH LIMITED METHANE LOSS AND LIMITED CO2 EMISSIONS

(30) Priorité: 13.05.2020 FR 2004692
(43) Date de publication de la demande: 22.03.2023
(73) Titulaire: L'AIR LIQUIDE, SOCIETE ANONYME POUR L'ETUDE ET L'EXPLOITATION DES PROCEDES GEORGES CLAUDE, 75007 Paris (FR)
(72) Inventeur: VALENTIN, Solène, 38360 Sassenage (FR); BARRAUD, François, 38360 Sassenage (FR)
(74) Mandataire: Air Liquide
(86) Numéro de dépôt international: PCT/EP2021/061656
(87) Numéro de publication internationale: WO 2021/228616

(56) Documents cités:
- EP-A1- 3 632 525
- US-A1- 2012 111 051
- US-A1- 2017 327 758
- US-A1- 2018 280 883

## Description

La présente invention est relative à une installation de traitement par perméation membranaire d'un courant gazeux contenant au moins du méthane et du dioxyde de carbone pour produire un courant gazeux riche en méthane et à un procédé mettant en œuvre une telle installation.

Elle concerne en particulier l'épuration de biogaz, dans le but de produire du biométhane conforme aux spécifications pour injection dans un réseau de gaz naturel.

Le biogaz est le gaz produit lors de la dégradation de matières organiques en l'absence d'oxygène (fermentation anaérobie) encore appelée méthanisation. Il peut s'agir d'une dégradation naturelle - on l'observe ainsi dans les marais ou les décharges d'ordures ménagères - mais la production de biogaz peut aussi résulter de la méthanisation de déchets dans un réacteur dédié, appelé méthaniseur ou digesteur.

De par ses constituants principaux - méthane et dioxyde de carbone - le biogaz est un puissant gaz à effet de serre ; il constitue aussi, parallèlement, une source d'énergie renouvelable appréciable dans un contexte de raréfaction des énergies fossiles.

Le biogaz contient majoritairement du méthane (CH4) et du dioxyde de carbone (CO2) dans des proportions variables en fonction du mode d'obtention mais également, en moindres proportions de l'eau, de l'azote, de l'hydrogène sulfuré, de l'oxygène, ainsi que des composés organiques autres, à l'état de traces.

Selon les matières organiques dégradées et les techniques utilisées, les proportions des composants diffèrent, mais en moyenne le biogaz comporte, sur gaz sec, de 30 à 75% de méthane, de 15 à 60% de CO2, de 0 à 15% d'azote, de 0 à 5% d'oxygène et des composés traces.

Le biogaz est valorisé de différentes manières. Il peut, après un traitement léger, être valorisé à proximité du site de production pour fournir de la chaleur, de l'électricité ou un mélange des deux (la cogénération); la teneur importante en dioxyde de carbone réduit son pouvoir calorifique, augmente les coûts de compression et de transport et limite l'intérêt économique de sa valorisation à cette utilisation de proximité.

Une purification plus poussée du biogaz permet sa plus large utilisation, en particulier, une purification poussée du biogaz permet d'obtenir un biogaz épuré aux spécifications du gaz naturel et qui pourra lui être substitué ; le biogaz ainsi purifié est le « biométhane ». Le biométhane complète ainsi les ressources de gaz naturel avec une partie renouvelable produite au coeur des territoires; il est utilisable pour exactement les mêmes usages que le gaz naturel d'origine fossile. Il peut alimenter un réseau de gaz naturel, une station de remplissage pour véhicules, il peut aussi être liquéfié pour être stocké sous forme de gaz naturel liquide (GNL)...

Les modes de valorisation du biométhane sont déterminés en fonction des contextes locaux : besoins énergétiques locaux, possibilités de valorisation en tant que biométhane carburant, existence à proximité de réseaux de distribution ou de transport de gaz naturel notamment. Créant des synergies entre les différents acteurs oeuvrant sur un territoire (agriculteurs, industriels, pouvoirs publics), la production de biométhane aide les territoires à acquérir une plus grande autonomie énergétique.

Plusieurs étapes doivent être franchies entre la collecte du biogaz et l'obtention du biométhane, produit final apte à être comprimé ou liquéfié.

En particulier, plusieurs étapes sont nécessaires avant le traitement qui vise à séparer le dioxyde de carbone pour produire un courant de méthane purifié. Une première étape consiste à comprimer le biogaz qui a été produit et acheminé à pression atmosphérique, cette compression peut être obtenue - de façon classique - via un compresseur. Les étapes suivantes visent à débarrasser le biogaz des composants corrosifs que sont le sulfure d'hydrogène et les composés organiques volatils (COV), les technologies utilisées sont de façon classique l'adsorption à pression modulée (PSA) et le piégeage sur charbon actif. Vient ensuite l'étape qui consiste à séparer le dioxyde de carbone pour disposer in fine de méthane à la pureté requise pour son usage ultérieur.

Le dioxyde de carbone est un contaminant typiquement présent dans le gaz naturel dont il est courant de devoir le débarrasser. Des technologies variées sont utilisées pour cela en fonction des situations ; parmi celles-ci, la technologie membranaire est particulièrement performante lorsque la teneur en CO2 est élevée ; elle est donc pour séparer le CO2 présent dans le biogaz, provenant des gaz de décharge ou des digesteurs de déchets végétaux ou animaux.

Les procédés membranaires de séparation de gaz utilisés pour la purification d'un gaz, qu'ils utilisent un ou plusieurs étages de membranes doivent permettre la production d'un gaz à la qualité requise, pour un faible coût, tout en minimisant les pertes du gaz que l'on souhaite valoriser. Ainsi, dans le cas de l'épuration du biogaz, la séparation effectuée est principalement une séparation CH4/CO2, devant permettre la production d'un gaz contenant en fonction de son utilisation plus de 85% de CH4, de préférence plus de 95% de CH4, plus préférentiellement plus de 97,5% de CH4, tout en minimisant les pertes de CH4 dans le gaz résiduaire et le coût d'épuration, ce dernier étant pour une part importante lié à la consommation électrique du dispositif de compression du gaz en amont des membranes.

La demande de brevet américain US 2012/111051 A1 divulgue un procédé d'élimination du dioxyde de carbone d'un flux d'alimentation contenant des hydrocarbures, utilisant une unité de séparation par membrane en conjonction avec un échangeur de chaleur et une unité de séparation du dioxyde de carbone.

Il est préférable que les installations permettant la production d'un flux gazeux enrichi en méthane puissent contrôler la perte de méthane.

Partant de là, un problème qui se pose est de fournir une installation permettant l'obtention d'un flux de biométhane à concentration constante sans perte de son composant principale le méthane.

Une solution de la présente invention est une installation pour le traitement par perméation membranaire d'un flux gazeux d'alimentation comprenant au moins du méthane et du dioxyde de carbone, ladite installation comprenant:
- une première unité de séparation par membrane apte à recevoir le flux gazeux d'alimentation et à fournir un premier perméat et un premier rétentat,
- une seconde unité de séparation par membrane apte à recevoir le premier rétentat et à fournir un second perméat et un second rétentat,
- un compresseur permettant de comprimer le premier perméat à une pression comprise entre 17 bar et 25 bar,
- Un moyen permettant de refroidir le premier perméat comprimé à une température inférieure à -40°C,
- Une colonne de distillation permettant de séparer le premier perméat refroidi en un flux gazeux et un flux liquide,
- Au moins un moyen permettant de recycler le flux gazeux sortant de la colonne de distillation à l'entrée de la première unité de séparation membranaire,
- un moyen de mesure de la concentration en méthane et/ou en dioxyde de carbone dans le flux gazeux sortant de la colonne de distillation,
- un moyen de comparaison de la concentration en méthane et/ou en dioxyde de carbone mesurée par le moyen de mesure avec une valeur cible, et
- un moyen d'ajustement de la pression et/ou de la température du premier perméat en fonction de la comparaison effectuée par le premier moyen de comparaison.

De préférence le moyen permettant de recycler le flux gazeux sortant de la colonne de distillation comprend un ensemble de vannes et de tuyaux.

Selon le cas l'installation selon l'invention peut présenter une ou plusieurs des caractéristiques ci-dessous :
- L'installation comprend un compresseur permettant de comprimer le flux gazeux d'alimentation à une pression comprise entre 8 bar et 16 bar en amont de la première unité de séparation membranaire.
- L'installation comprend un évaporateur permettant d'évaporer le flux liquide sortant de la colonne de distillation.
- L'installation comprend un réservoir de liquide permettant de stocker le flux liquide sortant de la colonne de distillation.
- L'installation comprend un moyen de purification du flux gazeux d'alimentation placé en amont de la première unité de séparation par membrane et permettant d'éliminer au moins en partie une impureté choisie parmi l'eau, le sulfure d'hydrogène et les composés organiques volatils.
- Le moyen permettant de refroidir le premier perméat comprimé à une température inférieure à -40°C comprend un ensemble de groupe froid et d'échangeurs thermiques.

La présente invention a également pour objet un procédé de traitement par perméation membranaire d'un flux gazeux d'alimentation comprenant au moins du méthane et du dioxyde de carbone, ledit procédé mettant en œuvre l'installation telle que définie ci-dessus et comprenant :
a) Une étape d'alimentation de la première unité de séparation membranaire par le flux gazeux d'alimentation de sorte à produire un premier rétentat enrichi en méthane par rapport au flux gazeux d'alimentation et un premier perméat enrichi en dioxyde de carbone par rapport au flux gazeux d'alimentation,
b) Une étape d'alimentation de la seconde unité de séparation membranaire par le premier rétentat de sorte à produire un second rétentat enrichi en méthane par rapport au premier rétentat et un second perméat enrichi en dioxyde de carbone par rapport au premier rétentat,
c) Une étape de compression du premier perméat à une pression comprise entre 17 bar et 25 bar,
d) Une étape de refroidissement du premier perméat comprimé à une température inférieure à -40°C,
e) Une étape de séparation du premier perméat refroidi en un flux gazeux et un flux liquide dans la colonne de distillation,
f) Une étape de recyclage du flux gazeux sortant de la colonne de distillation à l'entrée de la première unité de séparation membranaire,
g) Une étape de mesure de la concentration en méthane et/ou en dioxyde de carbone dans le flux gazeux sortant de la colonne de distillation,
h) Une étape de comparaison de la concentration en méthane et/ou en dioxyde de carbone mesurée par le moyen de mesure avec une valeur cible, et
i) Une étape d'ajustement de la pression et/ou de la température du premier perméat en fonction de la comparaison effectuée par le premier moyen de comparaison.

Notons que ce recyclage permet de recycler la totalité du CH4 contenu dans le flux gazeux. En effet, le flux gazeux sortant de la colonne de distillation comprendra essentiellement du méthane et le flux liquide sortant de la colonne de distillation comprendra essentiellement du dioxyde de carbone.

La partie recyclée dans le flux gazeux d'alimentation représentera de préférence 1/3 du flux gazeux d'alimentation.

Selon le cas, le procédé selon l'invention peut présenter une ou plusieurs des caractéristiques ci-dessous :
- De préférence des trois étapes g), h) et i) sont réalisées automatiquement par des moyens de transmission de données et de traitement de données. De préférence la valeur cible est comprise entre 10% de CH4 et 20% de CH4,
- Le procédé comprend une étape de compression du flux gazeux d'alimentation à une pression comprise entre 8 bar et 16 bar en amont de la première unité de séparation membranaire,
- Le procédé comprend une étape d'évaporation du flux liquide sortant de la colonne de distillation.
- Le procédé comprend une étape de stockage du flux liquide sortant de la colonne de distillation dans un réservoir liquide.
- Le procédé comprend une étape de purification du flux gazeux d'alimentation en amont de la première unité de séparation par membrane de manière à éliminer au moins en partie une impureté choisie parmi l'eau, le sulfure d'hydrogène et les composés organiques volatils,
- Le flux gazeux d'alimentation est du biogaz.

La solution proposée ici permet ici l'obtention d'un flux de biométhane à concentration constante sans perte de son composant principale le méthane mais permet également d'éviter les émissions de dioxyde de carbone.

## Revendications

1. Installation pour le traitement par perméation membranaire d'un flux gazeux d'alimentation comprenant au moins du méthane et du dioxyde de carbone, ladite installation comprenant:
- une première unité de séparation par membrane apte à recevoir le flux gazeux d'alimentation et à fournir un premier perméat et un premier rétentat,
- une seconde unité de séparation par membrane apte à recevoir le premier rétentat et à fournir un second perméat et un second rétentat,
- un compresseur permettant de comprimer le premier perméat à une pression comprise entre 17 bar et 25 bar,
- un moyen permettant de refroidir le premier perméat comprimé à une température inférieure à -40°C,
- une colonne de distillation permettant de séparer le premier perméat refroidi en un flux gazeux et un flux liquide,
- au moins un moyen permettant de recycler le flux gazeux sortant de la colonne de distillation à l'entrée de la première unité de séparation membranaire,
- un moyen de mesure de la concentration en méthane et/ou en dioxyde de carbone dans le flux gazeux sortant de la colonne de distillation,
- un moyen de comparaison de la concentration en méthane et/ou en dioxyde de carbone mesurée par le moyen de mesure avec une valeur cible, et
- un moyen d'ajustement de la pression et/ou de la température du premier perméat en fonction de la comparaison effectuée par le moyen de comparaison.

2. Installation selon la revendication 1, **caractérisée en ce qu'**elle comprend un compresseur permettant de comprimer le flux gazeux d'alimentation à une pression comprise entre 8 bar et 16 bar en amont de la première unité de séparation membranaire.

3. Installation selon l'une des revendications 1 ou 2, **caractérisée en ce qu'**elle comprend un évaporateur permettant d'évaporer le flux liquide sortant de la colonne de distillation.

4. Installation selon l'une des revendications 1 ou 2, **caractérisée en ce qu'**elle comprend un réservoir de liquide permettant de stocker le flux liquide sortant de la colonne de distillation.

5. Installation selon l'une des revendications 1 à 4, **caractérisée en ce qu'**elle comprend un moyen de purification du flux gazeux d'alimentation placé en amont de la première unité de séparation par membrane et permettant d'éliminer au moins en partie une impureté choisie parmi l'eau, le sulfure d'hydrogène et les composés organiques volatils.

6. Installation selon l'une des revendications 1 à 5, **caractérisée en ce que** le moyen permettant de refroidir le premier perméat comprimé à une température inférieure à -40°C comprend un ensemble de groupe froid et d'échangeurs thermiques.

7. Procédé de traitement par perméation membranaire d'un flux gazeux d'alimentation comprenant au moins du méthane et du dioxyde de carbone, ledit procédé mettant en œuvre l'installation telle que définie dans l'une des revendications 1 à 6 et comprenant :
a) Une étape d'alimentation de la première unité de séparation membranaire par le flux gazeux d'alimentation de sorte à produire un premier rétentat enrichi en méthane par rapport au flux gazeux d'alimentation et un premier perméat enrichi en dioxyde de carbone par rapport au flux gazeux d'alimentation,
b) Une étape d'alimentation de la seconde unité de séparation membranaire par le premier rétentat de sorte à produire un second rétentat enrichi en méthane par rapport au premier rétentat et un second perméat enrichi en dioxyde de carbone par rapport au premier rétentat,
c) Une étape de compression du premier perméat à une pression comprise entre 17 bar et 25 bar,
d) Une étape de refroidissement du premier perméat comprimé à une température inférieure à -40°C,
e) Une étape de séparation du premier perméat refroidi en un flux gazeux et un flux liquide dans la colonne de distillation,
f) Une étape de recyclage du flux gazeux sortant de la colonne de distillation à l'entrée de la première unité de séparation membranaire,
g) Une étape de mesure de la concentration en méthane et/ou en dioxyde de carbone dans le flux gazeux sortant de la colonne de distillation,
h) Une étape de comparaison de la concentration en méthane et/ou en dioxyde de carbone mesurée par le moyen de mesure avec une valeur cible, et
i) Une étape d'ajustement de la pression et/ou de la température du premier perméat en fonction de la comparaison effectuée par le moyen de comparaison.

8. Procédé selon la revendication 7, **caractérisé en ce qu'**il comprend une étape de compression du flux gazeux d'alimentation à une pression comprise entre 8 bar et 16 bar en amont de la première unité de séparation membranaire.

9. Procédé selon l'une des revendications 7 ou 8, **caractérisé en ce qu'**il comprend une étape d'évaporation du flux liquide sortant de la colonne de distillation.

10. Procédé selon l'une des revendications 7 à 9, **caractérisé en ce qu'**il comprend une étape de stockage du flux liquide sortant de la colonne de distillation dans un réservoir liquide.

11. Procédé selon l'une des revendications 7 à 10, **caractérisé en ce qu'**il comprend une étape de purification du flux gazeux d'alimentation en amont de la première unité de séparation par membrane de manière à éliminer au moins en partie une impureté choisie parmi l'eau, le sulfure d'hydrogène et les composés organiques volatils.

12. Procédé selon l'une des revendications 7 à 11, **caractérisé en ce que** le flux gazeux d'alimentation est du biogaz.

## Patentansprüche

1. Anlage zur Behandlung eines Speisegasstroms, der mindestens Methan und Kohlendioxid enthält, durch Membranpermeation, wobei die Anlage Folgendes umfasst:
- eine erste Membrantrenneinheit, die geeignet ist, den Speisegasstrom zu empfangen und ein erstes Permeat und ein erstes Retentat zu liefern,
- eine zweite Membrantrenneinheit, die geeignet ist, das erste Retentat aufzunehmen und ein zweites Permeat und ein zweites Retentat bereitzustellen,
- einen Kompressor, mit dem das erste Permeat auf einen Druck zwischen 17 bar und 25 bar komprimiert werden kann,
- ein Mittel zum Abkühlen des ersten komprimierten Permeats auf eine Temperatur unter -40°C,
- eine Destillationskolonne, mit der das erste gekühlte Permeat in einen Gasstrom und einen Flüssigkeitsstrom getrennt werden kann,
- mindestens ein Mittel, mit dem der aus der Destillationskolonne austretende Gasstrom zum Eingang der ersten Membrantrenneinheit zurückgeführt werden kann,
- ein Mittel zur Messung der Konzentration von Methan und/oder Kohlendioxid im Gasstrom, der aus der Destillationskolonne austritt,
- ein Mittel zum Vergleich der von dem Messmittel gemessenen Methan- und/oder Kohlendioxidkonzentration mit einem Zielwert und
- ein Mittel zum Einstellen des Drucks und/oder der Temperatur des ersten Permeats in Abhängigkeit von dem durch das Vergleichsmittel durchgeführten Vergleich.

2. Anlage nach Anspruch 1, **dadurch gekennzeichnet, dass** sie einen Kompressor umfasst, mit dem der Speisegasstrom stromaufwärts der ersten Membrantrenneinheit auf einen Druck zwischen 8 bar und 16 bar komprimiert werden kann.

3. Anlage nach einem der Ansprüche 1 oder 2, **dadurch gekennzeichnet, dass** sie einen Verdampfer umfasst, mit dem der aus der Destillationskolonne austretende Flüssigkeitsstrom verdampft werden kann.

4. Anlage nach einem der Ansprüche 1 oder 2, **dadurch gekennzeichnet, dass** sie einen Flüssigkeitstank umfasst, in dem der aus der Destillationskolonne austretende Flüssigkeitsstrom gespeichert werden kann.

5. Anlage nach einem der Ansprüche 1 bis 4, **dadurch gekennzeichnet, dass** sie ein Mittel zur Reinigung des Speisegasstroms umfasst, das vor der ersten Membrantrenneinheit angeordnet ist und es ermöglicht, zumindest teilweise eine Verunreinigung zu entfernen, die unter Wasser, Schwefelwasserstoff und flüchtigen organischen Verbindungen ausgewählt wird.

6. Anlage nach einem der Ansprüche 1 bis 5, **dadurch gekennzeichnet, dass** das Mittel zum Kühlen des ersten verdichteten Permeats auf eine Temperatur unter -40 °C eine Baugruppe aus Kühlaggregat und Wärmetauschern umfasst.

7. Verfahren zur Behandlung eines Speisegasstroms, der mindestens Methan und Kohlendioxid enthält, durch Membranpermeation, wobei das Verfahren die in einem der Ansprüche 1 bis 6 definierte Anlage einsetzt und Folgendes umfasst:
a) Ein Schritt, bei dem der erste Membrantrennungsabschnitt mit dem Zufuhrgasstrom beschickt wird, um ein erstes Retentat, das im Vergleich zum Zufuhrgasstrom mit Methan angereichert ist, und ein erstes Permeat, das im Vergleich zum Zufuhrgasstrom mit Kohlendioxid angereichert ist, zu erzeugen,
b) Ein Schritt, bei dem der zweiten Membrantrenneinheit das erste Retentat zugeführt wird, um ein zweites Retentat, das im Vergleich zum ersten Retentat mit Methan angereichert ist, und ein zweites Permeat, das im Vergleich zum ersten Retentat mit Kohlendioxid angereichert ist, zu erzeugen,
c) Ein Schritt zum Komprimieren des ersten Permeats auf einen Druck zwischen 17 bar und 25 bar,
d) Ein Schritt zum Kühlen des ersten komprimierten Permeats auf eine Temperatur unter -40 °C,
e) Ein Schritt zum Trennen des ersten gekühlten Permeats in einen Gasstrom und einen Flüssigkeitsstrom in der Destillationskolonne,
f) Ein Schritt zum Recyceln des aus der Destillationskolonne austretenden Gasstroms zum Einlass der ersten Membrantrenneinheit,
g) Ein Schritt zum Messen der Konzentration von Methan und/oder Kohlendioxid im Gasstrom, der die Destillationskolonne verlässt,
h) Ein Schritt zum Vergleichen der von der Messvorrichtung gemessenen Konzentration von Methan und/oder Kohlendioxid mit einem Sollwert, und
i) Ein Schritt zum Einstellen des Drucks und/oder der Temperatur des ersten Permeats in Abhängigkeit von dem von der Vergleichsvorrichtung durchgeführten Vergleich.

8. Verfahren nach Anspruch 7, **dadurch gekennzeichnet, dass** es einen Schritt umfasst, bei dem der Speisegasstrom stromaufwärts der ersten Membrantrenneinheit auf einen Druck zwischen 8 bar und 16 bar verdichtet wird.

9. Verfahren nach einem der Ansprüche 7 oder 8, **dadurch gekennzeichnet, dass** es einen Schritt des Verdampfens des aus der Destillationskolonne austretenden Flüssigkeitsstroms umfasst.

10. Verfahren nach einem der Ansprüche 7 bis 9, **dadurch gekennzeichnet, dass** es einen Schritt umfasst, bei dem der aus der Destillationskolonne austretende Flüssigkeitsstrom in einem Flüssigkeitsbehälter gespeichert wird.

11. **Verfahren** nach einem der Ansprüche 7 bis 10, **dadurch gekennzeichnet, dass** es einen Schritt zur Reinigung des Speisegasstroms stromaufwärts der ersten Membrantrenneinheit umfasst, um zumindest teilweise eine Verunreinigung zu entfernen, die aus Wasser, Schwefelwasserstoff und flüchtigen organischen Verbindungen ausgewählt ist.

12. Verfahren nach einem der Ansprüche 7 bis 11, **dadurch gekennzeichnet, dass** es sich bei dem zugeführten Gasstrom um Biogas handelt.

## Claims

1. Installation for the treatment by membrane permeation of a feed gas stream comprising at least methane and carbon dioxide, said installation comprising:
- a first membrane separation unit capable of receiving the feed gas stream and providing a first permeate and a first retentate,
- a second membrane separation unit capable of receiving the first retentate and supplying a second permeate and a second retentate,
- a compressor for compressing the first permeate to a pressure of between 17 bar and 25 bar,
- means for cooling the first compressed permeate to a temperature below -40°C,
- a distillation column to separate the first cooled permeate into a gaseous stream and a liquid stream,
- at least one means for recycling the gas stream leaving the distillation column to the inlet of the first membrane separation unit,
- means for measuring the concentration of methane and/or carbon dioxide in the gas stream leaving the distillation column,
- means for comparing the concentration of methane and/or carbon dioxide measured by the measuring means with a target value, and
- means for adjusting the pressure and/or temperature of the first permeate as a function of the comparison made by the comparison means.

2. Installation as claimed in claim 1, **characterised in that** it comprises a compressor for compressing the feed gas stream to a pressure of between 8 bar and 16 bar upstream of the first membrane separation unit.

3. Installation according to one of claims 1 or 2, **characterised in that** it comprises an evaporator for evaporating the liquid stream leaving the distillation column.

4. Installation according to one of claims 1 or 2, **characterised in that** it comprises a liquid tank for storing the liquid stream leaving the distillation column.

5. Installation according to one of claims 1 to 4, **characterised in that** it comprises a means of purifying the feed gas stream placed upstream of the first membrane separation unit and making it possible to eliminate at least in part an impurity chosen from water, hydrogen sulphide and volatile organic compounds.

6. Installation according to one of claims 1 to 5, **characterised in that** the means for cooling the first compressed permeate to a temperature below -40°C comprises an assembly of units comprising refrigeration and heat exchangers.

7. A process for treating a feed gas stream comprising at least methane and carbon dioxide by membrane permeation, said process using the installation as defined in one of claims 1 to 6 and comprising :
a) A step of feeding the first membrane separation unit with the feed gas stream so as to produce a first retentate enriched in methane relative to the feed gas stream and a first permeate enriched in carbon dioxide relative to the feed gas stream,
b) A step of feeding the first retentate to the second membrane separation unit so as to produce a second retentate enriched in methane relative to the first retentate and a second permeate enriched in carbon dioxide relative to the first retentate,
c) A step of compressing the first permeate to a pressure of between 17 bar and 25 bar,
d) A step of cooling the first compressed permeate to a temperature below -40°C,
e) A step for separating the first cooled permeate into a gaseous stream and a liquid stream in the distillation column,
f) A step of recycling the gas stream leaving the distillation column to the inlet of the first membrane separation unit,
g) A step of measuring the concentration of methane and/or carbon dioxide in the gas stream leaving the distillation column,
h) A step of comparing the concentration of methane and/or carbon dioxide measured by the measuring means with a target value, and
i) A step of adjusting the pressure and/or temperature of the first permeate as a function of the comparison carried out by the comparison means.

8. A process as claimed in claim 7, **characterised in that** it comprises a step of compressing the feed gas stream to a pressure of between 8 bar and 16 bar upstream of the first membrane separation unit.

9. Process according to one of claims 7 or 8, **characterized in that** it comprises a step of evaporating the liquid stream leaving the distillation column.

10. Process according to one of claims 7 to 9, **characterized in that** it comprises a step of storing the liquid stream leaving the distillation column in a liquid tank.

11. Process according to one of claims 7 to 10, **characterised in that** it comprises a step of purifying the feed gas stream upstream of the first membrane separation unit so as to remove at least in part an impurity selected from water, hydrogen sulphide and volatile organic compounds.

12. Process according to one of claims 7 to 11, **characterised in that** the feed gas stream is biogas.
